# EUROPÄISCHE PATENTANMELDUNG

(11) **EP 1 177 809 A1**
(43) Veröffentlichungstag der Anmeldung: **06.02.2002**
(21) Anmeldenummer: 00116761.8
(22) Anmeldetag: 03.08.2000
(51) Int. Cl.: A61M 16/04, A61B 1/267

(54) **Fiberoptisches Intubationsmandrin**

(71) Anmelder: Zimmer, Matthias, 18057 Rostock (DE)
(72) Erfinder: Zimmer, Matthias, 18057 Rostock (DE)

(57) **Zusammenfassung**

Die Intubation des Patienten ist unter normalen Umständen für einen erfahrenen Anästhesisten kein Problem. Unter besonderen Umständen allerdings ist es möglich, daß die Intubation sich schwieriger gestaltet.
Bisher werden für solche Intubationen klassische Bronchoskope benutzt, die relativ aufwendig zu installieren und zu bedienen sind. Unter Notfallbedingungen außerhalb der Klinik steht diese Technik nicht zur Verfügung.
Bisherige Entwürfe einer handlichen Intubationsfiberoptik erscheinen in ihrer Bedienung so komplex, das sie nicht einhändig zu bedienen sind oder sie lassen keine in situ-Einstellung der Krümmung des Tubusmandrins zur Anpassung an die individuelle Anatomie des Patienten zu.

Das Problem wird gelöst durch ein flexibles fiberoptisches Intubationsmandrin, das einhändig geführt und gesteuert werden kann.
Über die Einstellung der Krümmung des Mandrins unter Sichtkontrolle in situ wird die Anpassung an die vorliegende Anatomie zugelassen.
Die im Handgriff untergebrachte Steuerung ermöglicht die einhändige Bedienung des Gerätes.

## Beschreibung

### Technisches Problem der Erfindung

Die Intubation des Patienten ist unter normalen Bedingungen für einen erfahrenen Anästhesisten kein Problem. Unter besonderen Umständen allerdings ist es möglich, daß die Intubation sich schwieriger gestaltet.
Solche Fälle wären beispielsweise Patienten, bei denen eine direkte Sicht auf den Kehlkopfeingang nach Einstellung mittels Laryngoskop nicht möglich ist. Gründe dafür könnten Verletzungen und Ödeme im Gesicht oder Mund- und Rachenbereich sein, anatomisch-morphologische Besonderheiten und Retroflektionsstörungen des Halses. Unter diesen Bedingungen ist das Einführen des Tubus unter direkter Sicht mittels Laryngoskop erschwert, Verletzungen durch den intubierenden Arzt sind nicht auszuschließen und es ist eine fiberoptisch assistierte Intubation notwendig.
Bisher werden für solche Intubationen klassische Bronchoskope benutzt, die relativ aufwendig zu installieren und zu bedienen sind. Unter Notfallbedingungen außerhalb der Klinik steht diese Technik nicht zur Verfügung.
Bisherige Entwürfe einer handlichen Intubationsfiberoptik erscheinen in ihrer Bedienung so komplex, das sie entweder nicht einhändig zu bedienen sind oder sie lassen eine in situ-Einstellung der Krümmung des Tubusmandrins zur Anpassung an die individuelle Anatomie des Patienten nicht zu.

### Lösung des Problems

Das Problem wird gelöst durch ein flexibles fiberoptisches Intubationsmandrin, das einhändig geführt und gesteuert werden kann.
Über die Einstellung der Krümmung des Mandrins unter Sichtkontrolle in situ wird die Anpassung an die vorliegende Anatomie zugelassen.
Die im Handgriff untergebrachte Steuerung ermöglicht die einhändige Bedienung des Gerätes.

### Anwendungsgebiet

Der einfache Aufbau und die einfache Bedienung sollen den regelmäßigen Einsatz in der Anästhesie, Intensivmedizin und im Rettungsdienst ermöglichen.
Der intubierende Arzt kann den Tubus unter Sichtkonfrolle vorschieben. Dabei muß bei unzureichender Krümmung des Mandrins nicht der gesamte Tubus in seiner Position verändert werden, sondern die Achse des Mandrins wird so eingestellt, daß das tracheale Ende des Systems Tubus-Mandrin direkt vor dem Kehlkopfeingang positioniert und unter Sicht in die Trachea eingeführt wird. Nach dem Einführen des Tubus wird das Mandrin in üblicher Weise zurückgezogen.
Durch die aktive Krümmung des Tubus ist eine Druckbelastung auf die Zähne des Patienten annähernd auszuschließen und zur Intubation nur eine geringe Mundöffnung nötig. Die zusätzliche Sichtkontrolle könnte im Extremfall die Intubation sogar erst ermöglichen und dem Patienten im Notfall eine Koniotomie mit den möglichen Komplikationen ersparen. Zusätzlich ist durch die sichtkontrollierte Plazierung des Tubus proximal der Bifurcatio tracheae eine versehentliche zu tiefe Intubation mit nachfolgender Belüftung nur einer Lunge sicher auszuschließen.
Die Auslegung der Stellmechanik zur einhändigen Bedienung berücksichtigt die im Rettungswesen zu erwartenden Bedingungen (Intubation ohne assistierendes Personal).

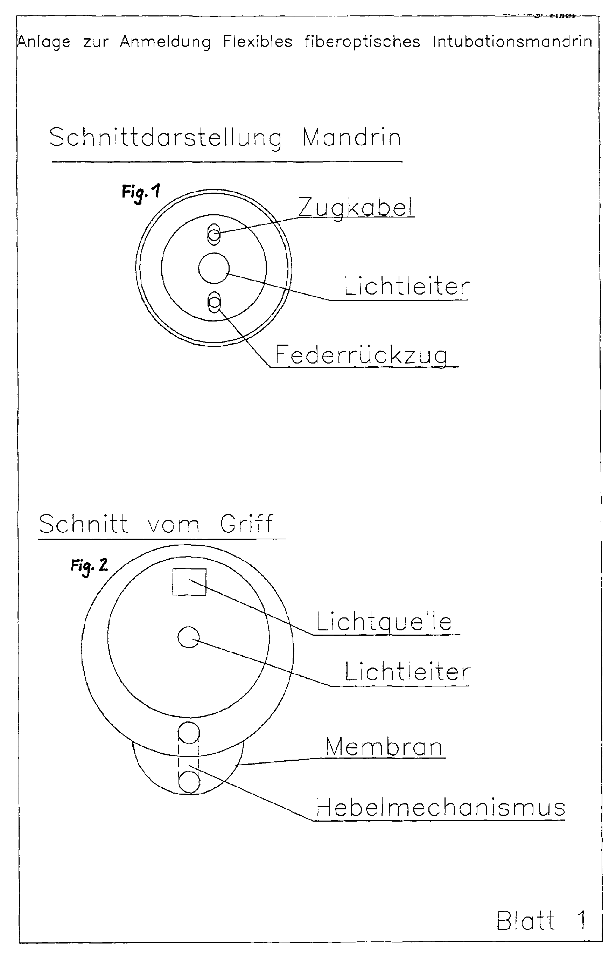

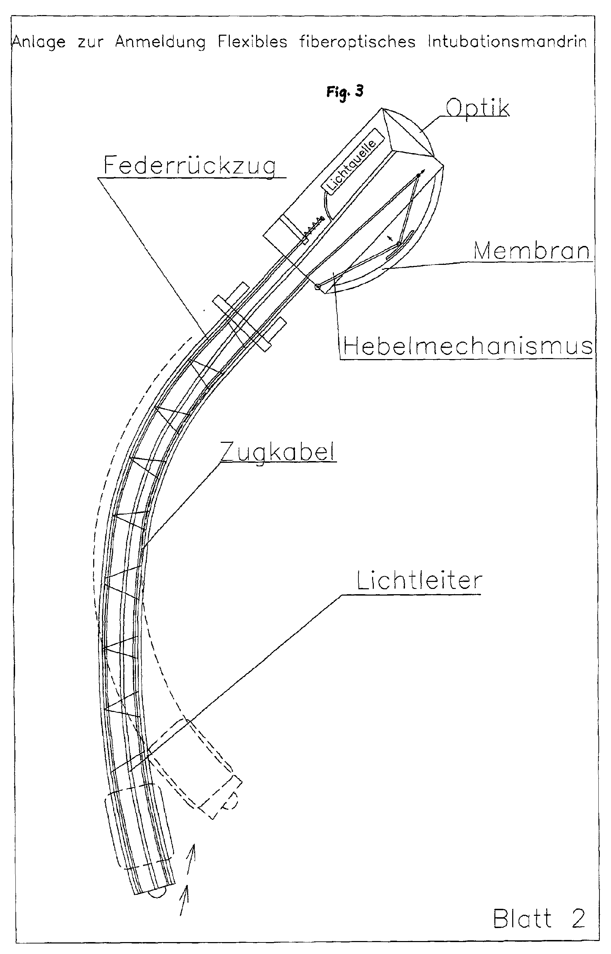

Die **Figuren 1 bis 3 der Zeichnung** zeigen Schnittdarstellungen des flexiblen fiberoptischen Intubationsmandrins in einer möglichen Ausführungsvariante

## Patentansprüche

1. Flexibles fiberoptisches Intubationsmandrin
**dadurch gekennzeichnet, daß**
- ein flexibles Hohlsystem durch eine Mechanik oder sonstige technische Vorrichtung( beispielsweise über Motoren) kontrolliert gekrümmt werden kann
- dieses Hohlsystem geeignet ist, eine Fiberoptik aufzunehmen und somit eine indirekte Sichtkontrolle aus der Position des trachealen Hohlsystemendes vom anderen Ende des Systems zuläßt
- der Außendurchmesser schmal genug ist und das Einführen in endotracheale Tuben ermöglicht
- die zur Krümmung benötigte Mechanik, wie auch das ganze Gerät, einhändig benutzt werden kann
- eine Variante mit und ohne eigene Lichtquelle benutzt werden kann
- das Gerät von einer den hygienischen Anforderungen genügenden Beschichtung umgeben ist.
